# EUROPÄISCHE PATENTSCHRIFT

(11) **EP 0 556 830 B1**
(45) Veröffentlichungstag und Bekanntmachung des Hinweises auf die Patenterteilung: **24.04.1996**
(21) Anmeldenummer: 93102566.2
(22) Anmeldetag: 18.02.1993
(51) Int. Cl.: C07D 311/58

(54) **Verfahren zur Herstellung von 6-Hydroxy-2,5,7,8-tetraalkyl-2-(4-aminophenoxymethyl)chromanen**
Method for the preparation of 6-hydroxy-2,5,7,8-tetraalkyl-2-(4-aminophenoxymethyl)chromans
Procédé pour la préparation des 6-hydroxy-2,5,7,8-tétraalkyl-2-(4-aminophénoxyméthyl)chromanes

(30) Priorität: 21.02.1992 CH 532/92
(43) Veröffentlichungstag der Anmeldung: 25.08.1993
(73) Patentinhaber: LONZA AG, CH-3945 Gampel/Wallis (CH); Sankyo Company Limited, Tokyo (JP)
(72) Erfinder: Heveling, Josef, Dr., Naters (Kanton Wallis) (CH)
(74) Vertreter: Weinhold, Peter, Dr.

(56) Entgegenhaltungen:
- EP-A- 0 207 581
- BULLETIN OF THE CHEMICAL SOCIETY OF JAPAN, Bd. 61, Nr. 9, Juni 1988, TOKYO, JP, Seiten 3283 - 3288 M. SHIBAGAKI, ET AL.: 'The catalytic reduction of aldehydes and ketones with 2-propanol over hydrous zirconium oxide'
- D2 : Chemistry Letters 1988, nr. 10, Seiten 1633-1636

## Beschreibung

Die Erfindung betrifft ein neues Verfahren zur Herstellung von 6-Hydroxy-2,5,7,8-tetraalkyl-2-(4-aminophenoxymethyl)chromanen (Aminochroman) der allgemeinen Formel 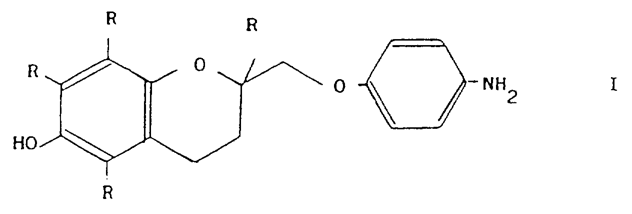 worin R eine Niederalkylgruppe mit 1 bis 4 C-Atomen bedeutet.

Diese Aminochromane der allgemeinen Formel I sind wertvolle Zwischenprodukte zur Herstellung von lipidsenkenden Pharmazeutika (J. Med. Chem. 1989, 32, S. 421)

Aus der EP-A 207 581 ist bekannt, Aminochromane gemäss der allgemeinen Formel I dadurch herzustellen, dass ein Tetraalkyl-2-(4-nitrophenoxymethyl)chroman-4-on zunächst mit Natriumborhydrid in das entsprechende Chroman-4-ol überführt wird, in einer weiteren Stufe das Chroman-4-ol in Gegenwart von p-Toluolsulfonsäure in das Chrom-3-en dehydratisiert wird und im letzten Schritt mit einem Hydrierkatalysator sowohl die Nitrogruppe als auch die Chromen-Doppelbindung zum Endprodukt hydriert wird.

Diese Umsetzung beinhaltet den Nachteil, dass zwischen den einzelnen Reaktionsschritten ein beträchtlicher Aufarbeitungsaufwand anfällt, der eine Umsetzung der Synthese in den technischen Masstab erschwert. Ausserdem ist die Reduktion mit Natriumborhydrid im Vergleich zu katalytischen Reduktionen teuer und aus ökologischer Sicht insofern problematisch, als dass die resultierenden Abwasser borbelastet sind.

Es stellte sich folglich die Aufgabe, eine einfachere Synthese zu entwickeln, die die genannten Nachteile nicht beinhaltet.

Die Aufgabe konnte erfindungsgemäss mit einem Verfahren gemäss Patentanspruch 1 gelöst werden.

Ausgegangen wird von den 6-Hydroxy-2,5,7,8-tetraalkyl-2-(4-nitrophenoxymethyl)chroman-4-onen (Nitrochromanon) der allgemeinen Formel 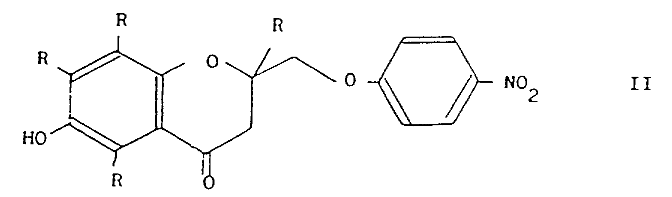 worin R die genannte Bedeutung hat.

Diese können z.B. gemäss EP-PS 139 421 aus Acetylhydrochinonderivaten hergestellt werden.
Vorzugsweise wird vom 6-Hydroxy-2,5,7,8-tetramethyl-2-(4-nitrophenoxymethyl)chroman-4-on (Formel II mit R = CH₃) ausgegangen.

Erfindungsgemäss wird in der ersten Stufe das genannte 6-Hydroxy-2,5,7,8-tetraalkyl-2-(4-nitrophenoxymethyl)chroman-4-on (Nitrochromanon) der allgemeinen Formel II in Gegenwart eines Hydrierkatalysators mit Wasserstoff zum 6-Hydroxy-2,5,7,8-tetraalkyl-2-(4-aminophenoxymethyl)chroman-4-on (Aminochromanon) der allgemeinen Formel 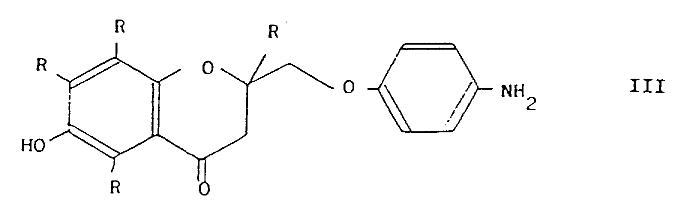 hydriert.

Als Hydrierkatalysatoren finden zweckmässig Edelmetallkatalysatoren, wie Platin oder Palladium, aufgebracht auf einen inerten Träger, wie z.B. Kohle oder Aluminiumoxid, Anwendung. Bevorzugt wird Palladium, aufgebracht in einer Menge von 0,5% bis 10% auf Kohle, eingesetzt.

Die Hydrierung erfolgt zweckmässig in einem niederen aliphatischen Alkohol, wie z.B. Ethanol oder Isopropanol, oder z.B. in Aromaten, wie Toluol, als Lösungsmittel. Isopropanol bietet den Vorteil, dass ein Lösungsmittelwechsel für die Folgestufe ausbleiben kann.

Zweckmässig wird bei Wasserstoffdrucken von 1 bar bis 20 bar, vorzugsweise 5 bar bis 10 bar und einer Temperatur zwischen 20°C und 100°C, vorzugsweise zwischen 40°C und 60°C, gearbeitet.

Die Wasserstoffaufnahme ist in der Regel nach 1 bis 2 h abgeschlossen, worauf zur Isolation des Aminochromanons gemäss Formel III der Katalysator abgetrennt und das Lösungsmittel abgedampft werden kann.

Gegebenenfalls kann nach Abtrennung des Katalysators die Reaktionslösung direkt für die Folgestufe eingesetzt werden.

In der zweiten Stufe wird erfindungsgemäss das Aminochromanon, gemäss Formel III, in Gegenwart des Katalysatorsystems amorphes Zirkonoxid/Isopropanol unter Druck zum 6-Hydroxy-2,5,7,8-tetraalkyl-2-(4-aminophenoxymethyl)chrom-3-en (Aminochromen) der allgemeinen Formel 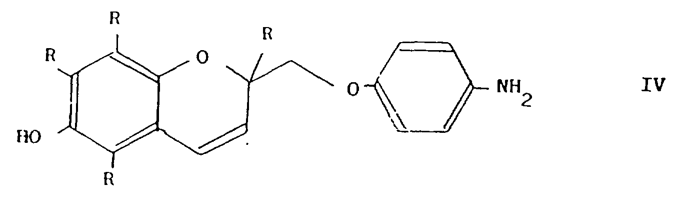 reduziert.

Das erfindungsgemäss eingesetzte amorphe Zirkonoxid kann auf bekannte Weise durch Fällung aus einer Zirkonylchloridlösung mit Natronlauge, gemäss Shibagaki et al. in Bull. Chem. Soc. Japn. 1988, 61, S. 3283ff (vgl. auch Schibagaki et al., Chem. Letters 1988, 1633), oder mit Ammoniak, gemäss US-PS 5 030 601, und anschliessender Trocknung und Kalzination, hergestellt werden.

Zweckmässig weisen die hergestellten amorphen Zirkonoxide eine spezifische Oberfläche nach BET zwischen 210 m/g und 265 m/g auf.
Bevorzugt wird ein amorphes Zirkonoxid eingesetzt, das durch Fällung mit Ammoniak erhalten wird.

Das aus dem Fällungsprozess erhaltene amorphe Zirkonoxid wird vor seinem Einsatz von Vorteil einer Vorbehandlung unterzogen.
Dies geschieht zweckmässig durch Behandlung des gefällten und kalzinierten amorphen Zirkonoxids in einer mobilen Inertgasatmosphäre bei 150°C bis 300°C über einen Zeitraum von 1 h bis 24 h.

Zweckmässig wird die Umsetzung in der zweiten Stufe so durchgeführt, dass das Aminochromanon gemäss Formel III zusammen mit dem vorbehandelten amorphen Zirkonoxid in Isopropanol, welches als Reaktionspartner und Lösungsmittel fungiert, vorgelegt wird.

Die Umsetzung erfolgt vorzugsweise unter Luftausschluss bei Drucken zwischen zweckmässig 1 bar bis 50 bar, vorzugsweise 20 bar bis 40 bar und einer Temperatur von zweckmässig zwischen 80°C bis 220°C, vorzugsweise 160°C bis 200°C.
Nach einer Reaktionsdauer von in der Regel 3 h bis 10 h ist die Umsetzung beendet.

Aus dem Reaktionsgemisch kann nach Abtrennen des Zirkonoxidkatalysators und Entfernen des Lösungsmittels das Aminochromen, gemäss Formel IV isoliert werden.

Es ist aber auch möglich die Reaktionslösung die das Aminochromen der Formel IV enthält nach Abtrennen des Zirkonoxidkatalysators direkt der Folgestufe zuzuführen.

In der letzten Stufe wird schliesslich das Aminochromen, der Formel IV, in Gegenwart eines Hydrierkatalysators mit Wasserstoff zum Endprodukt, der Formel I, hydriert.

Als Hydrierkatalysator finden zweckmässig Edelmetallkatalysatoren, wie Platin oder Palladium, aufgebracht auf einen inerten Träger, Anwendung.
Bevorzugt wird Palladium, aufgebracht in einer Menge von 0,5% bis 10% auf Kohle, eingesetzt.

Die Hydrierung erfolgt zweckmässig in einem niederen aliphatischen Alkohol, wie z.B. Isopropanol, oder in Aromaten wie Toluol, oder in einem Gemisch aus Toluol und einem der genannten Alkohole.

Zweckmässig wird bei Wasserstoffdrucken von 1 bar bis 20 bar, vorzugsweise 5 bar bis 10 bar, und einer Temperatur zwischen 15°C und 100°C, vorzugsweise bei 20°C bis 40°C hydriert.

Das Aminochroman der Formel I kann auf einfache Weise durch Abtrennen des Katalysators und Abtrennen des Lösungsmittels gewonnen werden.

### Beispiele

### A) Herstellung des amorphen Zirkonoxids

Zirkonylchloridoctahydrat (ZrOCl₂·8H₂O) wurde in Wasser gelöst. Die leichte Trübung wurde abfiltriert und mit entionisiertem Wasser auf einen Gehalt (ZrO₂) von 50 g/l eingestellt. Ein technischer Ammoniak (ca. 25%) wurde mit entionisiertem Wasser auf eine Konzentration von 10% verdünnt.

In einem Reaktionsgefäss wurden 2,5 1 entionisiertes Wasser vorgelegt. Unter Rühren bei 8000 U/min wurde die Zirkonylchloridlösung und die Ammoniaklösung kontrolliert zudosiert.

Die Dosierrate der ZrO₂-Lösung betrug 50 ml/min. Die Ammoniaklösung wurde so dosiert, dass während der resultierenden Fällung ein pH-Wert von 7,0 ± 0,2 eingehalten werden konnte.

Durch Zusatz von entionisiertem Wasser wurde der Feststoffgehalt der Suspension auf ca. 1% gehalten.
Nach beendeter Fällung wurde der Feststoff durch Filtration abgetrennt. Der Filterkuchen wurde mehrmals mit ammonakialischem Wasser solange gewaschen, bis der Cl⁻-Gehalt auf 0,05% gesunken war.
Der Filterkuchen wurde dann bei 100°C getrocknet, nochmals aufgeschlämmt, filtriert und erneut getrocknet.
Schliesslich wurde das erhaltene ZrO₂-Pulver während 8 h bei 300°C kalziniert. Das erhaltene ZrO₂ war röntgenamorph und hatte eine spezifische Oberfläche nach BET von 240 m/g.

### Beispiel 1

### a) Verfahren zur Herstellung von 6-Hydroxy-2,5,7,8-tetramethyl-2-(4-aminophenoxymethyl)-chroman-4-on (Aminochromanon III)

50 g (134,6 mmol) 6-Hydroxy-2,5,7,8-tetramethyl-2-(4-nitrophenoxymethyl)chroman-4-on (II) wurden mit 2,5 g eines Palladiumkatalysators auf Kohle (5% Pd/C) in 150 ml Isopropanol in einem Autoklaven vorgelegt.

Nach mehrmaligem Spülen mit Stickstoff und anschliessend mit Wasserstoff wurde bei einem Wasserstoffdruck von 5 bar und bei 50°C während 1,25 bis 1,5 h bei 750 U/min gerührt.
Das Reaktionsgemisch wurde anschliessend vom Katalysator befreit. Das Lösungsmittel wurde abgedampft.
Man erhielt 45,7 g (99,5%) des Titelproduktes. Gehalt (HPLC): 97%

### b₁) Verfahren zur Herstellung von 6-Hydroxy-2,5,7,8-tetramethyl-2-(4-aminophenoxymethyl)chrom-3-en (Aminochromen IV)

5 g (14,6 mmol) des Aminochromanons aus Stufe 1a) wurden mit 10 g amorphem Zirkonoxid (hergestellt gemäss A und vorbehandelt während 2 h bei 200°C unter Argondurchfluss) in 100 g trockenem Isopropanol in einem Autoklaven unter Luftausschluss vorgelegt.
Nach mehrmaligem Spülen mit Stickstoff wurde unter Rühren bei 750 U/min, bei einem Druck von 10 bar auf eine Temperatur von 190°C erwärmt. Der Druck stieg dabei auf 27 bis 31 bar. Nach 8 h wurde die Reaktionsmischung auf Raumtemperatur abgekühlt und das amorphe Zirkonoxid abgetrennt. Die Reaktionslösung enthielt gemäss GC das Aminochromen (IV) in einer Ausbeute von 85,3% und das Aminochroman (I) in einer Ausbeute von 4,7%.

### b₂) Verfahren zur Herstellung von 6-Hydroxy-2,5,7,8-tetramethyl-2-(4-aminophenoxymethyl)chrom-3-en (Aminochromen IV)

Die Umsetzung wurde entsprechend b₁ aber in Gegenwart von 80 g Isopropanol und 20 g Toluol durchgeführt.
Nach Abtrennung des Lösungsmittels wurde 4,8 g Produkt gewonnen. Davon entfielen 87,1% auf das Aminochromen (IV) und 3,4% auf das Aminochroman (I).

### c₁) Verfahren zur Herstellung von 6-Hydroxy-2,5,7,8-tetramethyl-2-(4-aminophenoxymethyl)chroman (Aminochroman I)

Die im Beispiel b₁) erhaltene Lösung enthaltend 85,3% des Aminochromens (IV) wurden mit 0,4 g eines Palladiumkatalysators auf Kohle (5% Pd/C) und 25 g Toluol in einem Autoklaven vorgelegt.
Nach mehrmaligem Spülen mit Stickstoff und anschliessend mit Wasserstoff, wurde bei einem Wasserstoffdruck von 8 bar und bei Raumtemperatur (24 - 27°C) während 1,5 h bei 750 U/min gerührt.
Das Reaktionsgemisch wurde vom Katalysator befreit. Das Lösungsmittel wurde abgedampft.
Das Produkt (4,6 g) enthielt das Titelprodukt in einer Ausbeute von 81,6 %.

### c₂) Verfahren zur Herstellung von 6-Hydroxy-2,5,7,8-tetramethyl-2-(4-aminophenoxymethyl)chroman (Aminochroman I)

Die Umsetzung wurde entsprechend c₁) aber mit 4,8 g des Produktes aus b₂) enthaltend 87,1% des Aminochromens IV in Gegenwart von 46,1 g Toluol und 5,4 g Methanol als Lösungsmittel durchgeführt.
Das Rohprodukt (4,6 g) enthielt das Titelprodukt in einer Ausbeute von 92,8% (GC).

## Patentansprüche

1. Verfahren zur Herstellung von 6-Hydroxy-2,5,7,8-tetraalkyl-2-(4-aminophenoxymethyl)-chromanen der allgemeinen Formel 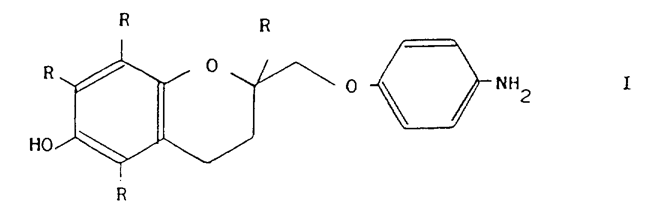 worin R eine C₁-C₄-Alkylgruppe bedeutet, dadurch gekennzeichnet, dass ein 6-Hydroxy-2,5,7,8 -tetraalkyl-2-(4-nitrophenoxymethyl)-chroman-4-on der allgemeinen Formel 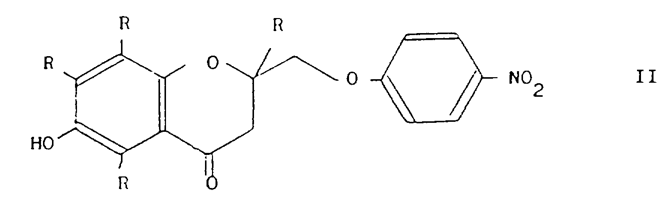 worin R die genannte Bedeutung hat, in der ersten Stufe in Gegenwart eines Hydrierkatalysators mit Wasserstoff zum 6-Hydroxy-2,5,7,8-tetraalkyl-2-(4-aminophenoxymethyl)-chroman-4-on der allgemeinen Formel 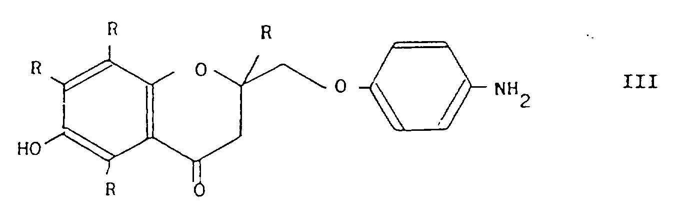 hydriert wird, in einem zweiten Stufe dieses Aminochromanon in Gegenwart des Katalysatorsystems amorphes Zirkonoxid/Isopropanol unter Druck zum 6-Hydroxy-2,5,7,8-tetraalkyl-2-(4-aminophenoxymethyl)-chrom-3-en der allgemeinen Formel 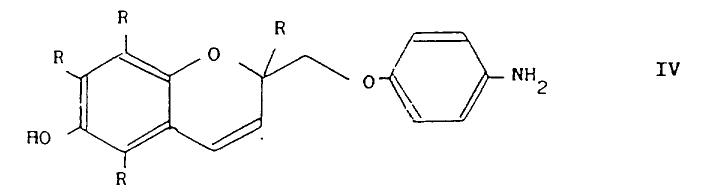 reduziert wird und dieses Aminochromen schliesslich in der dritten Stufe in Gegenwart eines Hydrierkatalysators mit Wasserstoff in das Endprodukt überführt wird.

2. Verfahren nach Patentanspruch 1, dadurch gekennzeichnet, dass die Hydrierung in der ersten Stufe mit einem Platin- oder Palladiumkatalysator, aufgebracht auf einen inerten Träger, durchgeführt wird.

3. Verfahren nach Patentanspruch 2, dadurch gekennzeichnet, dass Palladium, aufgebracht in einer Menge von 0,5 bis 10% auf Kohle, eingesetzt wird.

4. Verfahren nach einem der Patentansprüche 1 bis 3, dadurch gekennzeichnet, dass die Hydrierung in der ersten Stufe bei einem Wasserstoffdruck von 1 bis 20 bar und einer Temperatur zwischen 20°C und 100°C durchgeführt wird.

5. Verfahren nach einem der Patentansprüche 1 bis 4, dadurch gekennzeichnet, dass die Reduktion in der zweiten Stufe bei einem Druck von 1 bis 50 bar und einer Temperatur zwischen 80°C und 220°C durchgeführt wird.

6. Verfahren nach Patentanspruch 5, dadurch gekennzeichnet, dass die Reduktion in der zweiten Stufe bei einem Druck von 20 bis 40 bar und einer Temperatur zwischen 160°C und 200°C durchgeführt wird.

7. Verfahren nach einem der Patentansprüche 1 bis 6, dadurch gekennzeichnet, dass für die Reduktion ein amorphes Zirkonoxid eingesetzt wird, das durch ammoniakalische Fällung einer Zirkonylchloridlösung und durch anschliessende Trocknung und Kalzination des gefällten Zirkonoxids hergestellt wird.

8. Verfahren nach Patentanspruch 7, dadurch gekennzeichnet, dass das amorphe Zirkonoxid einer Vorbehandlung in einer mobilen Inertgasatmosphäre bei einer Temperatur zwischen 150°C und 300°C unterzogen wird.

9. Verfahren nach einem der Patentansprüche 1 bis 8, dadurch gekennzeichnet, dass als Hydrierkatalysator für die Hydrierung in der dritten Stufe ein Platin- oder Palladiumkatalysator, aufgebracht auf einen inerten Träger, verwendet wird.

10. Verfahren nach Patentanspruch 9, dadurch gekennzeichnet, dass Palladium, aufgebracht in einer Menge von 0,5% bis 10% auf Kohle, verwendet wird.

11. Verfahren nach einem der Patentansprüche 1 bis 10, dadurch gekennzeichnet, dass die Hydrierung in der dritten Stufe bei einem Wasserstoffdruck von 1 bis 20 bar und einer Temperatur von 15°C bis 100°C durchgeführt wird.

## Claims

1. A process for producing 6-hydroxy-2,5,7,8-tetraalkyl-2-(4-aminophenoxymethyl)-chromans of the general formula: 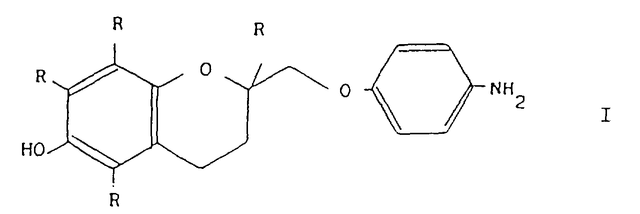 wherein R is a C₁ to C₄ alkyl group, characterized in that a 6-hydroxy-2,5,7,8-tetraalkyl-2-(4-nitrophenoxymethyl)-chroman-4-one of the general formula: 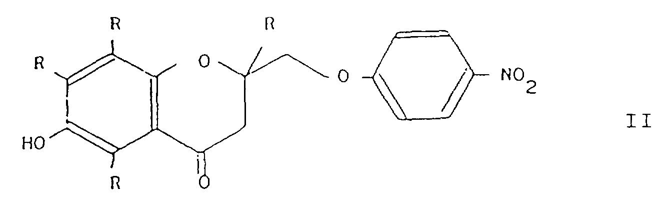 wherein R has the above meaning, is hydrogenated in the first step in the presence of a hydrogenation catalyst with hydrogen to a 6-hydroxy-2,5,7,8-tetraalkyl-2-(4-aminophenoxymethyl)-chroman-4-one of the general formula 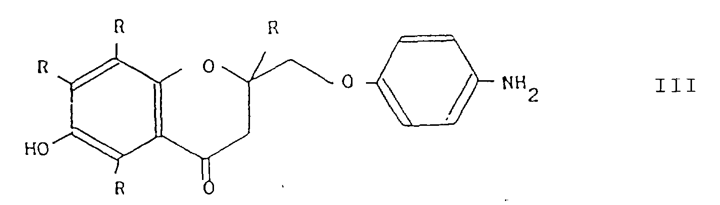 said aminochromanone being then reduced in a second step under pressure in the presence of a catalyst system of amorphous zirconium oxide/isopropanol to a 6-hydroxy-2,5,7,8-tetraalkyl-2-(4-aminophenoxymethyl)-chrom-3-ene of the general formula: 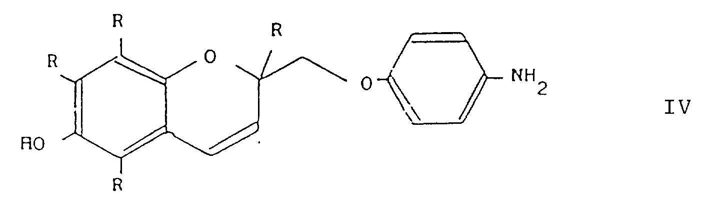 and said aminochromene being finally converted in a third step in the presence of a hydrogenation catalyst with hydrogen into the final product.

2. The process according to Claim 1, characterized in that hydrogenation in the first step is conducted with a platinum or palladium catalyst applied to an inert substrate.

3. The process according to Claim 2, characterized by using palladium applied in an amount of from 0.5 to 10% to carbon.

4. The process according to any one of Claims 1 to 3, characterized in that hydrogenation in the first step is conducted at a hydrogen pressure of from 1 to 20 bar and a temperature between 20° and 100°C.

5. The process accordingt to any one of Claims 1 to 4, characterized in that reduction in the second step is conducted at a pressure of from 1 to 50 bar and a temperature between 80° and 220°C.

6. The process according to Claim 5, characterized in that reduction in the second step is conducted at a pressure of from 20 to 40 bar and a temperature between 160° and 200°C.

7. The process according to any one of Claims 1 to 6, characterized by using for the reduction an amorphous zirconium oxide prepared by precipitating with ammonia a zirconyl chloride solution and subsequently drying and calcining the precipitated zriconium oxide.

8. The process according to Claim 7, characterized by subjecting the amorphous zirconium oxide to a pretreatment in a mobile inert gas atmopshere at a temperature between 150° to 300°C.

9. The process according to any one of Claims 1 to 8, characterized by using as hydrogenation catalyst for the hydrogenation in the third step a platinum or palladium catalyst applied to an inert substrate.

10. The process according to Claim 9, characterized by using palladium applied in an amount of from 0.5 to 10% to carbon.

11. The process according to any one of Claims 1 to 10, characterized in that hydrogenation in the third step is conducted at a hydrogen pressure of from 1 to 20 bar and a temperature of from 15° to 100°C.

## Revendications

1. Procédé pour la préparation des 6-hydroxy-2,5,7,8-tétraalkyl-2-(4-aminophénoxyméthyl)-chromanes de la formule générale 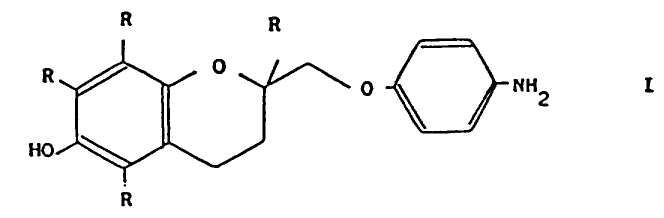 dans laquelle R signifie un groupe alkyle C₁-C₄, caractérisé en ce que l'on hydrogène une 6-hydroxy-2,5,7,8-tétraalkyl-2-(4-nitrophénoxyméthyl)-chromane-4-one de la formule générale 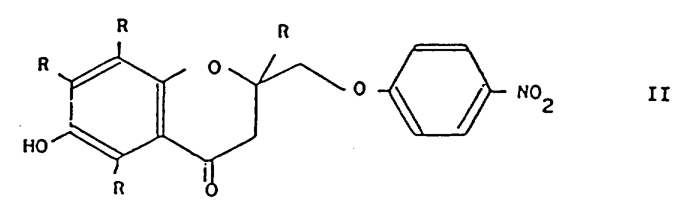 dans laquelle R a la signification indiquée, dans la première étape en présence d'un catalyseur d'hydrogénation avec de l'hydrogène en 6-hydroxy-2, 5,7, 8-tétraalkyl-2-(4-aminophénoxyméthyl)-chromane-4-one de la formule générale 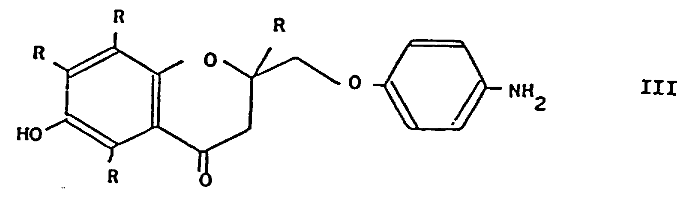 dans une deuxième étape cette aminochromanone est réduite en présence du système catalyseur oxyde de zirconium amorphe/isopropanol sous pression en 6-hydroxy-2,5,7,8-tétraalkyl-2-(4-aminophénoxyméthyl)-chrom-3-ène de la formule générale 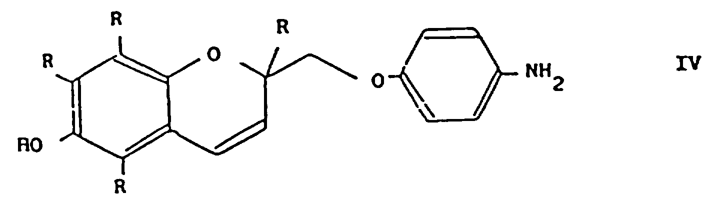 et cet aminochromène est finalement transformé dans la troisième étape en présence d'un catalyseur d'hydrogénation avec de l'hydrogène en le produit final.

2. Procédé selon la revendication 1, caractérisé en ce que l'hydrogénation s'effectue dans la première étape avec un catalyseur au platine ou au palladium, appliqué sur un support inerte.

3. Procédé selon la revendication 2, caractérisé en ce que l'on met en oeuvre du palladium appliqué dans une quantité de 0,5 à 10 % sur du carbone.

4. Procédé selon l'une des revendications 1 à 3, caractérisé en ce que l'hydrogénation s'effectue dans la première étape sous une pression d'hydrogène de 1 à 20 bars et à une température entre 20°C et 100°C.

5. Procédé selon l'une des revendications 1 à 4, caractérisé en ce que la réduction dans la deuxième étape s'effectue sous une pression de 1 à 50 bars et à une température entre 80°C et 220°C.

6. Procédé selon la revendication 5, caractérisé en ce que la réduction dans la deuxième étape s'effectue à une pression de 20 à 40 bars et à une température entre 160°C à 200°C.

7. Procédé selon l'une des revendications 1 à 6, caractérisé en ce que pour la réduction, on met en oeuvre un oxyde de zirconium amorphe qui est préparé par précipitation ammoniacale d'une solution de chlorure de zirconyle et par séchage consécutif et calcination de l'oxyde de zirconium précipité.

8. Procédé selon la revendication 7, caractérisé en ce que l'oxyde de zirconium amorphe est soumis à un prétraitement dans une atmosphère de gaz inerte mobile à une température entre 150°C et 300°C.

9. Procédé selon l'une des revendications 1 à 8, caractérisé en ce que l'on utilise comme catalyseur d'hydrogénation pour l'hydrogénation dans la troisième étape un catalyseur au platine ou au palladium appliqué sur un support inerte.

10. Procédé selon la revendication 9, caractérisé en ce que l'on utilise du palladium appliqué dans une quantité de 0,5 % à 10 % sur du carbone.

11. Procédé selon l'une des revendications 1 à 10, caractérisé en ce que l'hydrogénation dans la troisième étape s'effectue à une pression d'hydrogène de 1 à 20 bars et à une température de 15°C à 100°C.
